# EUROPEAN PATENT SPECIFICATION

(11) **EP 1 915 129 B1**
(45) Date of publication and mention of the grant of the patent: **28.10.2009**
(21) Application number: 06762809.9
(22) Date of filing: 25.07.2006
(51) Int. Cl.: A61K 9/08, A61K 31/4704, A61K 47/12

(54) **PHARMACEUTICAL FORMULATIONS COMPRISING A LONG-ACTING BETA2-AGONIST FOR ADMINISTRATION BY NEBULISATION**
PHARMAZEUTISCHE FORMULIERUNGEN MIT EINEM LANGZEIT-BETA-2-AGONISTEN ZUR VERABREICHUNG DURCH VERNEBLUNG
FORMULATIONS PHARMACEUTIQUES COMPRENANT UN AGONISTE BETA2 A LONGUE DUREE D'ACTION ADMINISTREES PAR NEBULISATION

(30) Priority: 01.08.2005 EP 05016664
(43) Date of publication of application: 30.04.2008
(73) Proprietor: CHIESI FARMACEUTICI S.p.A., I-43100 Parma (IT)
(72) Inventor: SOLIANI RASCHINI, Annamaria, I-43100 Parma (IT); BODRIA, Alessandro, I-43100 Parma (IT)
(74) Representative: Minoja, Fabrizio
(86) International application number: PCT/EP2006/007322
(87) International publication number: WO 2007/014673

(56) References cited:
- EP-A- 1 452 179
- WO-A-2005/013994
- WO-A-2006/105401
- WO-A2-02/083079
- US-A1- 2004 023 935

## Description

The invention relates to a liquid, propellant-free pharmaceutical formulation in the form of ready-to-use preparation for administration by nebulisation comprising a water soluble salt of the beta₂-agonist 8-hydroxy-5-[1-hydroxy-2-[[2-(4-methoxyphenyl)-1-methylethyl]amino]ethyl]-2(1H)-quinolinone as active ingredient. The active ingredient is chemically stable in the formulation, and said formulation is provided of an adequate shelf-life suitable for commercial distribution, storage and use.

### BACKGROUND OF THE INVENTION

Airway obstruction characterizes a number of sever respiratory diseases including asthma and chronic obstructive pulmonary disease (COPD).

In particular asthma is a disease becoming more and more prevalent and is the most common disease of childhood.

Since the most important factor in its treatment is to achieve relaxation of the bronchial smooth muscle cells, compounds having beta₂-adrenoreceptor agonist activity (hereinafter beta₂-agonists) are the drugs of choice for treating such a condition.

These beta₂-agonists include compounds belonging to the class of phenylalkylamino derivatives of the first generation such as salbutamol, procaterol, fenoterol and terbutalin and of second generation (long-acting beta₂-agonists) such as formoterol {2-hydroxy-5-((1RS)-1-hydroxy-2-(((1RS)-2-(p-methoxyphenyl)-1-methylethyl)amino)ethyl)formanilide)} and salmeterol, which overcome the disadvantage of the short duration of action particularly for patients with nocturnal asthma.

Beta₂-agonists are currently administered by pulmonary delivery which relies on inhalation of an aerosol through the mouth and throat so that the drug substance can reach the lungs. One of the advantages of the inhalatory route over the systemic one is the possibility of delivering the drug directly to the site of action, avoiding any systemic side-effects, thus resulting in a more rapid clinical response and a higher therapeutic index.

The drug can be administered as a liquid (aqueous or hydroalcoholic) formulation through a nebuliser, as a dry powder by means of a Dry Powder Inhaler (DPIs) or in a halogenated hydrocarbon propellant which requires a suitable pressurized metered-dose inhaler (pMDIs) releasing a metered dose of medicine upon each actuation.

Liquid formulations, in particular aqueous formulations, are easy to administer as they are inhaled during normal breathing through a mouth-piece or a face-mask. Therefore they are particularly suitable for young and elderly people who are most often the patients in need of such therapy and who experience difficulties using other devices.

8-Hydroxy-5-[1-hydroxy-2-[[2-(4-methoxyphenyl)-1-methylethyl] amino]ethyl]-2(1H)-quinolinone of formula I

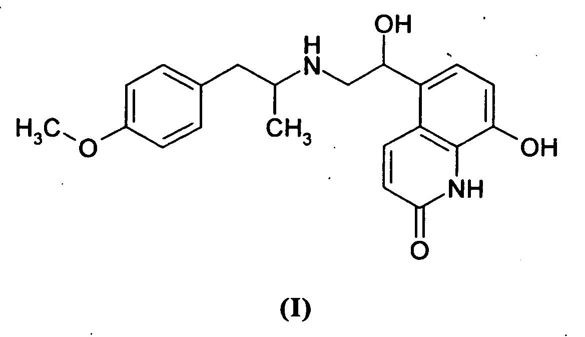

The quinolinone compound of formula I may be in the form of a mixture of four stereoisomers, or in the form of a mixture of two stereoisomers, such as a mixture of the (R)(R)- and the (R)(S)-stereoisomers (wherein the term (R)(S)- stereoisomer means that the asymmetric carbon atom at the position -CH(CH3)- has (R) configuration and the asymmetric carbon atom at the position -CH(OH)- has (S) configuration). In a preferred embodiment the quinolinone (I) is in the form of the pure (R)(R)-enantiomer (hereinafter indicated with the recommended International Nonproprietary Name (INN) carmoterol) which is a highly potent long-acting beta₂-agonist also characterized by a rapid onset of action, disclosed for the first time in EP 147719.

In the prior art its hydrochloride salt has also been referred to as TA 2005 or CHF 4226.

From a chemical point of view, carmoterol is a 2(1H)-quinolinone/carbostyril derivative characterised by the presence of a 8-hydroxy-2(1H)-quinolinone/carbostyryl ring and a 1-hydroxy-2-[2-(4-methoxyphenyl)-1-methylethyl]amino]ethyl side chain.

As other beta₂-agonists, carmoterol may suffer from problems of chemical stability in solution. In particular carmoterol undergoes oxidation of the hydroxyl group present on the alkylamino side chain of the molecule. The oxidation, which is followed by the cleavage of the molecule, is mainly catalysed by molecular oxygen and/or heavy metal ions impurities such as ferric ions which are present in the solution.

On the other hand, its chemical structure may account for a different degradation rate and for more and different mechanisms of decomposition with respect to other phenylalkylamino derivatives.

Moreover, carmoterol is endowed with a particularly high potency, therefore it is formulated at a very low concentration. It is well known that more diluted is the active ingredient in the solution, the higher are the chemical stability problems.

Therefore it would be highly advantageous to find out suitable conditions for providing liquid propellant-free, in particular aqueous formulations, wherein carmoterol would be chemically stable.

### THE PRIOR ART

EP 147719 first disclosed carbostyril derivatives and salts thereof whose general formula carmoterol belongs to. Only generic information was reported about the formulations.

EP 1157689 and WO 2005/084640 deal with aerosol pharmaceutical solution formulations to be used with pressurized metered dose inhalers (MDIs) wherein beta₂-agonists belonging to the class of phenylalkylamino derivatives such as TA 2005 and formoterol in solution in a HFA propellant and a co-solvent are stabilized by adding a mineral acid.

WO2005/013994 and WO2005/013945 in the name of Boehringer concern pharmaceutical compositions to be administered by inhalation comprising a beta₂-agonist whose formula corresponds to carmoterol, or an enantiomer thereof, in combination with anticholinergic agents and steroids, respectively.

Only a broad and generic teaching is provided concerning formulations suitable for inhalation. As far as propellant free inhalable formulations are concerned, many kinds of possible formulations containing a wide range of excipients are proposed.

The pH can be adjusted by using either inorganic or organic acids.

Preferred inorganic acids are hydrochloric and sulfuric acids, while ascorbic acid, fumaric acid, and citric acid are the preferred organic acids.

Particularly preferred is the use hydrochloric acid.

WO 02/083079 ('079) in the name of Dey discloses formulations in the form of a solution of bronchodilating agents such as beta₂-agonists in a pharmacologically suitable fluid that contains water, that are stable during long term storage.

The compositions can contain a buffer. A long list of possible buffers is provided. In one embodiment the buffer is citric acid/phosphate buffer, acetate buffer, citrate buffer or phosphate buffer. In another embodiment, the buffer is a citrate buffer (citric acid/sodium citrate).

Enabling disclosure and specific compositions are only given for formoterol:
in particular the inventors stated that its kinetic-pH profile is dependent on buffer concentration and, at low and approximately neutral conditions, the increase of the buffer concentration from 5 mM to 20 mM increased its rate constant of decomposition significantly. On the other hand they also observed that no noticeable differences in rate constant were observed in the pH region of about 4.5 to about 5.5 with increasing buffer concentration from 5 mM to 20 mM.

TA 2005 is only generically cited among a long list bronchodilating agents.

In Chen et al (J Pharm Sci 1987, 76, 703-706), the kinetics of degradation of the carbostyril derivative procaterol was investigated.

The effect of pH, temperature and ferric ions on the reaction rate was evaluated.

Solutions at pH 4.0, 4.5, 5.0 and 5.5 were prepared using an acetate buffer, while a solution at pH 6.0 was prepared by adding sodium acetate and monobasic sodium phosphate.

Procaterol in solution was more stable at more acidic pHs and protected form air. The lowest degradation rate was obtained at pH 4.0.

The presence of ferric ions increased the auto-oxidation rate of procaterol and diminished the duration of its induction period with the exception of the solution at pH 6.0 where a mixed buffer of acetate and phosphate was used. The authors suggested that, being phosphate a good metal-complexing agent, may bind the trace quantity of ferric ion, thus the metal becomes unavailable for oxidative catalytic action.

In summary, some of the documents of the prior art offer a very limited and generic disclosure, others propose different possible and alternative solutions, while few of them seem to indicate that each beta₂-agonist requires specific conditions to be stabilised in an aqueous solution.

In particular, as far as 2(1H)-quinolinone/carbostyril derivatives such as CHF 4226 are concerned, the prior art provides an enabling teaching only for formulations comprising a HFA propellant.

### SUMMARY OF THE INVENTION

The present invention provides a liquid, propellant-free pharmaceutical formulation in the form of ready-to-use preparation for administration by nebulisation, comprising:
i) a physiologically acceptable water soluble salt of 8-hydroxyl-5-[1-hydroxy-2-[[2-(4-methoxyphenyl)-1-methylethyl]amino]ethyl]-2(1H)-quinolinone which may be in the form of a mixture of four stereoisomers, or in the form of a mixture of two stereoisomers, such as a mixture of the (R)(R)- and the (R)(S)-stereoisomers (wherein the term (R)(S)-stereoisomer means that the configuration at the asymmetric carbon atom at the position -CH(CH3)- has (R) configuration and the asymmetric carbon atom at the position -CH(OH)- has (S) configuration). In a preferred embodiment the quinolinone (I) is in the form of the pure (R)(R)-enantiomer (carmoterol) as active ingredient;
ii) a solvent selected from water or an aqueous solution comprising at least 50% v/v of water and a co-solvent miscible with water;
iii) a buffering agent
wherein the pH of the solution is comprised between 4.0 and 5.0 and the buffering agent comprises citric acid.

The physiologically acceptable water soluble salt of carmoterol is advantageously present in a concentration corresponding to a percentage w/v of carmoterol free base comprised between 0.0001 and 0.004%.

The invention is also directed to a kit comprising the pharmaceutical formulation provided herein and a nebulizer.

The invention is further directed to the use of the pharmaceutical formulation provided herein for the manufacture of a medicament for the prevention or treatment of diseases characterized by reversible airway obstruction such as asthma or chronic obstructive airways disease (COPD).

### DEFINITIONS

As used herein, the term 'water soluble' refers to a solute which is soluble in water according to the European Pharmacopoeia II Ed 5.2 2005, page 565, e.g. that needs 3 ml of solvent for dissolving 100 mg of solute.

As used herein the terms 'chemically stable' means that the compound in the formulation exhibits substantial chemical stability over time. Preferably it refers to a formulation wherein the active ingredient is stable according to the requirements of the ICH Guideline Q1A referring to "Stability Testing of new Active Substances (and Medicinal Products), e.g. when the change in its assay after 6 months under accelerated conditions (40°C, 75% R.H.) is less than 5% from its initial value [R.H. = relative humidity].

As used herein, the term 'ready-to-use preparation for administration by nebulisation' refers to a preparation which is administered directly without further handling and is dispersed in air to form an aerosol by means of a nebulizer, e.g. an instrument that is capable of generating very fine liquid droplet for inhalation into the lungs.

The formulation of the invention can be realized in a lyophilised form in unitary doses for the reconstitution in a solution. In this alternative embodiment a single dose of a lyophilised preparation may be reconstituted before use with a solvent vial in a solution.

### DETAILED DESCRIPTION OF THE INVENTION

It is an object of the invention to provide a liquid propellant-free pharmaceutical formulation, in the form of ready-to-use preparation for administration by nebulisation, comprising 8-hydroxy-5-[1-hydroxy-2-[[2-(4-methoxyphenyl)-1-methylethyl]amino]ethyl-2(1H)-quinolinone as active ingredient, wherein the active ingredient is chemically stable and said formulation being provided of an adequate shelf-life suitable for commercial distribution, storage and use.

The quinolinone compound may be in the form of a mixture of four stereoisomers, or in the form of a mixture of two stereoisomers, such as a mixture of the (R)(R)- and the (R)(S)-stereoisomers (wherein the term (R)(S)-stereoisomer means that the asymmetric carbon atom at the position -CH(CH3)- has (R) configuration and the asymmetric carbon atom at the position -CH(OH)- has (S) configuration) and more preferably in the form of the pure (R)(R)-enantiomer (carmoterol).

There is provided a liquid, propellant-free pharmaceutical formulation in the form of ready-to-use preparation for administration by nebulisation, comprising:
i) aphysiologically acceptable water soluble salt of 8-hydroxy-5-[(1R)-1-hydroxy-2-[[(1R)-2-(4-methoxyphenyl)-1-methylethyl]amino]ethyl]-2(1H)-quinolinone (carmoterol) as active ingredient;
ii) a solvent selected from water or an aqueous solution comprising at least 50% v/v of water and a co-solvent miscible with water;
iii) a buffering agent
wherein the pH of the solution is comprised between 4.0 and 5.0 and the buffering agent comprises citric acid.

In case of a lyophilised preparation the buffering agent and one or more optional carrier, diluent or solubilising agents may be added before carrying out the lyophylisation.It has indeed been found that in a liquid propellant-free formulation, carmoterol is provided with an adequate stability for pharmaceutical use by adjusting the pH to a very narrow range e.g. comprised between 4.0 to 5.0 with a very specific kind of buffering agents, e.g. those comprising citric acid.

Stability studies in different conditions of pH were carried out.

As water soluble salt, the carmoterol hydrochloride salt, referred to as CHF 4226, was utilised.

The main degradation products forming during storage turned out be the two compounds deriving from the oxidation of the hydroxyl group present on the alkylamino side chain of carmoterol, followed by cleavage of the molecule, e.g. 5-formyl-8-hydroxy-2(1H)-quinolinone, hereinafter referred to as CHF 1756, and *p*-methoxy-amphetamine hereinafter referred to as PMA.

The amount of the main degradation products and the total impurities and degradation products of CHF 4226 were determined by HPLC.

Total impurities and degradation products comprise the starting impurities present in the drug, the main degradation products and other minor degradation products forming during storage.

In a first study, the effect of the pH in a rather broad range comprised between 3.0 and 6.0 adjusted with different kind of agents was investigated.

The different formulations were stored under long term (25°C, 60% R.H.) and accelerated (40°C, 75% R.H.) conditions for three months.

CHF 4226 turned out be chemically more stable in an aqueous solution adjusted to a pH of 4.5 ± 0.1 with a citrate buffer, e.g. a mixture of citric acid and sodium citrate, at a concentration of about 19 mM.

In particular, after three months under accelerated conditions, CHF 4226 in said formulation gave rise to an amount of total impurities/degradation products of less than 1.3% by weight.

Under the same conditions, formulations having the same pH but adjusted with different buffering agents other than citrate, i.e. phosphate, acetate or ascorbate buffers, showed an amount of total impurities/degradation products higher than 3.5% 6%, and 27% by weight, respectively.

On the other hand, at the same pH value of 4.5, formulations whose pH was adjusted with inorganic acids such as hydrochloric, sulfuric or phosphoric acid were not stable giving rise to an amount of total impurities/degradation products higher than 5% after 1 month under accelerated conditions. An analogous behavior was observed for formulations containing phosphoric acid or phosphoric acid/phosphate buffer having a pH of 3.0 and 6.0, respectively.

The chemical stability of CHF 4226 was then investigated in the pH interval comprised from 4.0 to 5.5 adjusted with buffers comprising citric acid at different concentrations.

The different formulations were stored under accelerated conditions (40°C, 75% R.H.) for at least four months.

It has been found that particular buffers, and in particular buffers comprising citric acid enhanced the chemical stability of CHF 4226 in liquid, propellant-free formulations. It has also been found that the chemical stability of CHF 4226 improved in the pH interval between 4.0 and 5.0, preferably between 4.0 and 4.5.

Moreover, it has been found that the chemical stability of CHF 4226 seems to be affected by the concentration of the buffer.

The amount of total impurities/degradation indeed appeared to increase over time more slowly for the formulation adjusted to pH 4.5 with a citrate buffer concentration of 10 mM in comparison to the formulation adjusted with a citrate buffer concentration of about 17 mM.

The aforementioned findings were observed for the hydrochloride salt.

However, since the main degradation products occurs through oxidation of the hydroxyl group, any physiologically acceptable salt of carmoterol may be used for the purposes of the invention, provided that it is water soluble according to the European Pharmacopoeia II Ed 5.2 2005, page 565.

Advantageously, suitable salts of carmoterol, include salts with inorganic acids, such as hydrochloride, hydrobromide, phosphate, and sulphate salts and salts with organic acids, such as salicylate, citrate, tartrate and mandelate salts.

In a preferred embodiment, the formulation of the invention contains the hydrochloride salt of carmoterol referred to as CHF 4226.

Advantageously the concentration of the physiologically acceptable water soluble salt of carmoterol corresponds to a percentage w/v of carmoterol free base comprised between 0.0001 and 0.004%, preferably between 0.0003 and 0.002%, more preferably between 0.0005 and 0.001 %.

In the pharmaceutical formulations of the invention, the physiologically acceptable water soluble salt of carmoterol is dissolved in water or in an aqueous solution comprising at least 50% v/v of water and a co-solvent, miscible with water. Said co-solvent includes, but it is not limited to, polar compounds that contain one or more hydroxyl groups or other polar groups. For example, it includes alcohols, such as ethanol, isopropanol, and glycols including propylene glycol, polyethylene glycol, polypropylene glycol, glycol ether, glycerol and polyoxyethylene alcohols. In one embodiment of the invention, the preferred co-solvent is propylene glycol.

Preferably the aqueous solution includes at least 60% v/v of water, more preferably at least 80% v/v of water.

In one of the preferred embodiments of the invention, the formulation comprises only water as a solvent.

The formulations herein provided shall have a pH comprised between 4.0 and 5.0, preferably between 4.0 and 4.5 and shall be adjusted with a buffering agent comprising citric acid including, but not limited to the citric acid/sodium citrate couple (e.g. citrate buffer) and the citric acid/disodium phosphate couple. Citric acid and sodium citrate may be used in the form of hydrates.

The suitable buffer composition in terms of ratio between the acid and the salt, necessary for achieving the claimed pH interval of the invention may be determined empirically using methods well known to those skilled in the art.

Those skilled in the art are also aware of the fact that the experimental pH value may vary of ± 0.1 units.

The buffer concentration for use herein can advantageously vary from 0.1 mM to 20 mM, preferably from 0.5 to 18 mM, more preferably between 1 to 15 mM, even more preferably between 2 and 10 mM. In one of the embodiment of the invention, the preferred buffer concentration is comprised between 1 and 10 mM. In other embodiments, the buffer concentration may be comprised between 5 and 10 mM or between 1 and 5 mM.

In the formulations of the invention tonicity adjusting agents such as sodium chloride may be added to provide an osmolarity ranging between 250 and 450 mOsm/l, preferably between 260 and 400, even more preferably between 280 and 350 mOsm/l.

The pharmaceutical formulations of the invention could also contain an additional active ingredient and in particular a corticosteroid or an antimuscarinic/anticholinergic drug suspended or dissolved in the solution. Examples of a corticosteroid are: beclomethasone dipropionate, fluticasone propionate, mometasone furoate, triamcinolone acetonide, budesonide and its 22R-epimer, ciclesonide and rofleponide. Examples of antimuscarinic/anticholinergic drugs are ipratropium bromide, oxitropium bromide, tiotropium bromide, and glycopyrrolate bromide.

If the additional active ingredient is one which is chemically unstable in the pH conditions of the present invention, is preferably formulated as a suspension.

In the case of steroids, these are preferably used in suspended form, in particular if the solvent used is only water.

However, if a suitable co-solvent is added, the steroid may also be present in the form of a solution. For example, budesonide is sufficiently soluble if dissolved in a mixture of water and propylene glycol.

In general terms, the person skilled in the art, on the basis of the available information concerning the stability as well as the water solubility of the active ingredient to be used in combination, shall suitably select those whose chemical stability is compatible with the pH and type of buffer of the formulation of the invention as well as adjust the percentage of the co-solvent in the aqueous solution in order to achieve their complete dissolution.

The formulation of the invention may be distributed in suitable containers such as multidose vials or, preferably, unit dose vials for single dosage administration. Said unit-dose vials may be pre-sterilised or, preferably, may be aseptically filled using the "blow, fill and seal" technology. The filling is preferably carried out under inert atmosphere. Solution formulations can be advantageously sterilized by filtration.

The unit-dose vials are preferably of 2 ml.

The formulations of the invention are intended for administration by nebulisation using suitable apparatus such as jet nebulisers, ultrasonic nebulisers, soft-mist nebulisers such as Respimat^{®} or others.

Therefore the invention is also directed to a kit comprising the pharmaceutical formulation provided herein filled in vials for single dosage administration and a nebulizer.

The invention is further directed to the use of the pharmaceutical formulations provided herein for the manufacture of a medicament for the prevention or treatment of diseases characterized by reversible airway obstruction such as asthma or chronic obstructive airways disease (COPD) including chronic bronchitis and emphysema, bronchiolitis, bronchiectasis and exacerbation of airways hyperreactivity consequent to other drug therapy.

The invention is illustrated by the following examples.

### EXAMPLES

### Example 1

### Preparation of a 0.001% w/v CHF 4226 propellant-free liquid formulation using a citrate buffer

To a 2 1 vessel, 3.6 g citric acid monohydrate, 5.2 g sodium citrate dihydrate, and 15.4 g sodium chloride were added. Purified water (1700 ml) was added to the vessel and the contents were mixed under magnetic stirring for 10 minutes at 500 r.p.m.

CHF 4226 (20 mg) was added and the solution was further stirred for 30 minutes at 1000 r.p.m. The obtained solution was brought to the final volume of 2 1 with purified water, filtered through a 0.2 µm Nylon filter, and distributed in 2 ml unit dose vials under nitrogen purging.

The composition for 2 ml unit-dose vial is reported in Table 1.

**Table 1 - Composition of the formulation**

| **Ingredient** | **Quantity (mg)** | **Concentration (% w/v)** |
|---|---|---|
| CHF 4226 | 0.02 | 0.001 |
| Citric acid monohydrate | 3.6 | 0.18 |
| Sodium citrate dihydrate | 5.2 | 0.26 |
| Sodium chloride | 15.4 | 0.77 |
| Purified water | q.s. to 2 ml | |

The pH of the solution turned out to be 4.48 and the osmolarity of 283-287 mOsm/kg.

### Example 2

### Stability studies carried out on the formulation of Example 1

The stability of the formulation filled in the 2 ml vials was evaluated both under long-term (25°C, 60% R.H.) and accelerated conditions (40°C, 75% R.H.) [R.H. = relative humidity]. The amount of CHF 1756, PMA and total impurities/degradation products of CHF 4226, expressed as percentage by weight, were determined by HPLC.

The total impurities and degradation products comprise the starting impurities present in the drug, the main degradation products CHF 1756 and PMA and other minor degradation products forming during storage.

The formulation of the invention turned out to be stable for at least 3 months under both long-term and accelerated conditions.

After three months under long-term conditions an amount of 0.29% by weight of total impurities/degradation products was detected, while after three months under accelerated, conditions the amount was less than 1.3% by weight.

### Example 3

### Stability of CHF 4226 in aqueous solution in the pH interval comprised from 4.0 to 5.5 adjusted with buffers comprising citric acid at different concentrations

Various liquid, propellant-free formulations comprising 0.001% w/v CHF 4226 were prepared according the procedure described in the Example 1 except that sodium chloride was not added in order to better appreciate the effect of the buffer concentration on the chemical stability.

The pH was adjusted in the interval comprised between 4.0 and 5.5 with buffers consisting of different relative percentages of the citric acid/sodium citrate couple or the citric acid/dibasic sodium phosphate couple. Each formulation was distributed in 20 ml glass vials.

The stability of the formulations was evaluated under accelerated conditions (40°C, 75% R.H.) for at least four months.

The impurities/degradation products of CHF 4226 were determined by HPLC using the experimental conditions reported in the Example 2.

The composition of the formulations in terms of pH, and type/concentration of the buffer as well as the results in terms of degradation products expressed as percentage by weight are reported in Table 2.

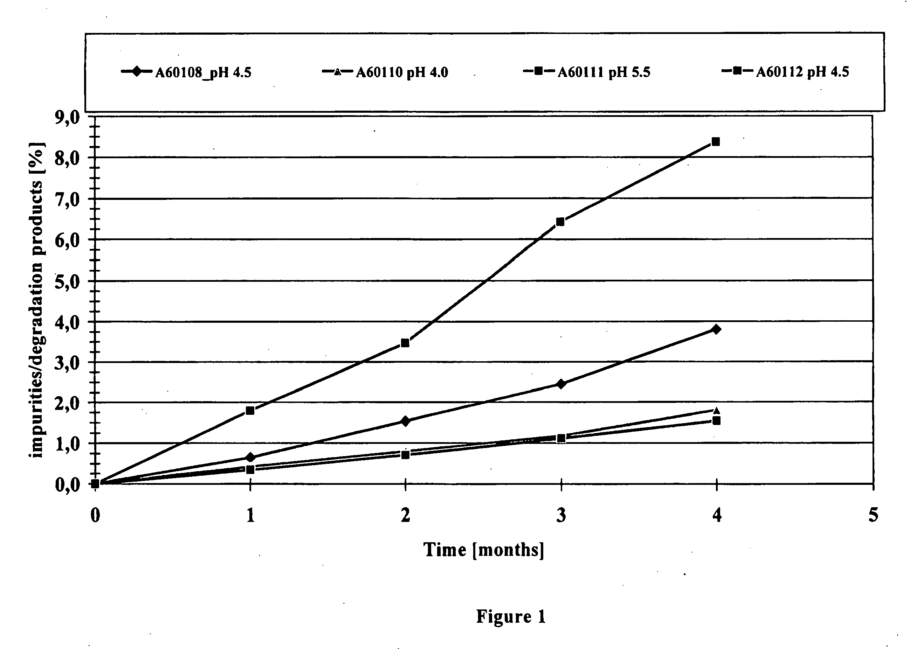

The variation of the amount of total impurities/degradation products after 1, 2, 3 and 4 months is reported in Figure 1.

The results demonstrate that the formulations comprising CHF 4226 exhibits an improved chemical stability at a pH comprised between 4.0 and 4.5 in comparison to those at pH 5.5. In fact, after four months, in the case of the former formulations, an amount of total impurities/degradation products significantly lower than 5% by weight (e.g. ranging from 1.5 to 3.8% by weight), whereas the latter ones gave rise to an amount higher than 5% by weight.

The results also indicate that the chemical stability of CHF 4226 appears to be affected by the concentration of the buffer.

As it can be clearly appreciated from the Figure 1, after 4 months of storage, the amount of total impurities/degradation increases over time more slowly for the formulation adjusted to pH 4.5 with a citrate buffer concentration of 10 mM in comparison to the formulation adjusted with a citrate buffer concentration of about 17 mM.

**Table 2 - Composition of the formulations in terms of pH, and type/concentration of the buffer and the results in terms of degradation products expressed as percentage by weight.**

| **Formulation** | **pH** | **buffer** | **buffer conc (mM)** | **PMA (% by weight)** | **CHF 1756 (% by weight)** | **Total degradation products (% by weight)** |
|---|---|---|---|---|---|---|
| A60110 | 4.0 | citric acid/sodium citrate | 10 | 1.04 | 0.59 | 1.80 |
| A60112 | 4.5 | citric acid/disodium phosphate | 10,5 | 0.85 | 0.36 | 1.54 |
| A60108 | 4.5 | citric acid/sodium citrate | 17,4 | 1.82 | 0.91 | 3.80 |
| A60111 | 5.5 | citric acid/sodium citrate | 10 | 3.83 | 1.22 | 8.37 |

## Claims

1. A liquid, propellant-free pharmaceutical formulation in the form of ready-to-use preparation for administration by nebulisation, comprising:
i) a physiologically acceptable water soluble salt of 8-hydroxy-5-[(1R)-1-hydroxy-2-[[(1R)-2-(4-methoxyphenyl)-1-methylethyl]amino]ethyl]-2(1H)-quinolinone (carmoterol) as active ingredient;
ii) a solvent selected from water or an aqueous solution comprising at least 50% v/v of water and a co-solvent miscible with water;
iii) a buffering agent
wherein the pH of the solution is comprised between 4.0 and 5.0 and the buffering agent comprises citric acid.

2. The pharmaceutical formulation according to claim 1 wherein the salt of carmoterol is selected from the group consisting of salts with inorganic acids, such as hydrochloride, hydrobromide, phosphate, and sulphate salts and salts with organic acids, such as salicylate, citrate, tartrate and mandelate salts.

3. The pharmaceutical formulation according to claim 2 wherein the salt is the hydrochloride salt, referred to as CHF 4226.

4. The pharmaceutical formulation according to any one of claims 1 to 3 wherein the concentration of the water soluble salt of carmoterol corresponds to a percentage w/v of carmoterol free base comprised between 0.0001 and 0.004%.

5. The pharmaceutical formulation according to claim 4 wherein the concentration of the salt is comprised between 0.0003 and 0.002%.

6. The pharmaceutical formulation according to claim 5 wherein the concentration of the salt is comprised between 0.0005 and 0.001%.

7. The pharmaceutical formulation according to any one of the preceding claims wherein the pH of the solution is comprised between 4.0 and 4.5.

8. The pharmaceutical formulation according to any one of preceding claims, wherein the buffering agent is selected from the group consisting of citric acid/sodium citrate (citrate buffer) or citric acid/disodium phosphate couple.

9. The pharmaceutical formulation according to claim 8 wherein the buffering agent is present in a concentration comprised between 0.1 and 20 mM.

10. The pharmaceutical formulation according to claim 9 wherein the concentration is comprised between 1 and 15 mM.

11. The pharmaceutical formulation according to claim 10 wherein the concentration is comprised between 2 and 10 mM.

12. The pharmaceutical formulation according to any one of the preceding claims wherein the solvent comprises only water.

13. The pharmaceutical formulation according to claim 12 further containing a tonicity adjusting agent in order to provide an osmolarity ranging from 250 and 450 mOsm/l.

14. The pharmaceutical formulation according to claim 13 wherein the tonicity adjusting agent is sodium chloride.

15. The pharmaceutical formulation according to any one of the claims 1 to 11 wherein the co-solvent is a polar compound that contains one or more hydroxyl groups.

16. The pharmaceutical formulation according to claim 15 wherein the polar compound is propylene glycol.

17. The pharmaceutical formulation according to any of the preceding claims further comprising an additional active ingredient suspended or dissolved in the solvent.

18. The pharmaceutical formulation according to claim 17 wherein the additional active ingredient is a corticosteroid.

19. The pharmaceutical formulation according to claim 17 wherein the additional active ingredient is an antimuscarinic/anticholinergic drug.

20. Use of the pharmaceutical formulation of any one of the preceding claims for the manufacture of a medicament for the prevention or treatment of a disease **characterized by** reversible airway obstruction.

21. The use according to claim 20 wherein the disease is asthma or chronic obstructive airways disease (COPD).

22. A kit comprising:
a) a pharmaceutical formulation according to any one of claims 1 to 19 filled in vials for single dosage administration; and
b) a nebulizer.

## Patentansprüche

1. Flüssige, Treibmittel-freie pharmazeutische Formulierung in der Form einer gebrauchsfertigen Zubereitung zur Verabreichung durch Vernebelung, umfassend:
i) ein physiologisch verträgliches, wasserlösliches Salz von 8-Hydroxy-5-[(1R)-1-hydroxy-2-[[(1R)-2-(4-methoxyphenyl)-1-methylethyl]amino]ethyl]-2(1H)-chinolinon (Carmoterol) als aktives Ingredienz;
ii) ein Lösungsmittel, ausgewählt aus Wasser und einer wässerigen Lösung, die wenigstens 50% V/V Wasser und ein mit Wasser mischbares Co-Lösungsmittel umfasst;
iii) ein Puffermittel,
wobei der pH der Lösung zwischen 4,0 und 5,0 liegt und das Puffermittel Citronensäure umfasst.

2. Pharmazeutische Formulierung gemäß Anspruch 1, wobei das Salz von Carmoterol ausgewählt ist aus der Gruppe, bestehend aus Salzen mit anorganischen Säuren, zum Beispiel Hydrochlorid-, Hydrobromid-, Phosphat- und Sulfatsalzen, und Salzen mit organischen Säuren, zum Beispiel Salicylat-, Citrat-, Tartrat- und Mandelatsalzen.

3. Pharmazeutische Formulierung gemäß Anspruch 2, wobei das Salz das Hydrochloridsalz ist, das als CHF 4226 bezeichnet wird.

4. Pharmazeutische Formulierung gemäß einem der Ansprüche 1 bis 3, wobei die Konzentration des wasserlöslichen Salzes von Carmoterol einem G/V-Prozentgehalt an Carmoterol als freie Blase, der zwischen 0,0001 und 0,004% liegt, entspricht.

5. Pharmazeutische Formulierung gemäß Anspruch 4, wobei die Konzentration des Salzes zwischen 0,0003 und 0,002% liegt.

6. Pharmazeutische Formulierung gemäß Anspruch 5, wobei die Konzentration des Salzes zwischen 0,0005 und 0,001% liegt.

7. Pharmazeutische Formulierung gemäß einem der vorangehenden Ansprüche, wobei der pH der Lösung zwischen 4,0 und 4,5 liegt.

8. Pharmazeutische Formulierung gemäß einem der vorangehenden Ansprüche, wobei das Puffermittel aus der Gruppe, bestehend aus dem Citronensäure/Natriumcitrat-Paar (Citratpuffer) und dem Citronensäure/Dinatriumphosphat-Paar, ausgewählt ist.

9. Pharmazeutische Formulierung gemäß Anspruch 8, wobei das Puffermittel in einer Konzentration vorliegt, die zwischen 0,1 und 20 mM liegt.

10. Pharmazeutische Formulierung gemäß Anspruch 9, wobei die Konzentration zwischen 1 und 15 mM liegt.

11. Pharmazeutische Formulierung gemäß Anspruch 10, wobei die Konzentration zwischen 2 und 10 mM liegt.

12. Pharmazeutische Formulierung gemäß einem der vorangehenden Ansprüche, wobei das Lösungsmittel nur Wasser umfasst.

13. Pharmazeutische Formulierung gemäß Anspruch 12, die außerdem ein die Tonizität einstellendes Mittel enthält, um eine Osmolarität im Bereich von 250 und 450 mOsm/l bereitzustellen.

14. Pharmazeutische Formulierung gemäß Anspruch 13, wobei das die Tonizität einstellende Mittel Natriumchlorid ist.

15. Pharmazeutische Formulierung gemäß einem der Ansprüche 1 bis 11, wobei das Co-Lösungsmittel eine polare Verbindung ist, die eine Hydroxylgruppe oder mehrere Hydroxylgruppen enthält.

16. Pharmazeutische Formulierung gemäß Anspruch 15, wobei die polare Verbindung Propylenglykol ist.

17. Pharmazeutische Formulierung gemäß einem der vorangehenden Ansprüche, die außerdem ein zusätzliches aktives Ingredienz in dem Lösungsmittel suspendiert oder gelöst umfasst.

18. Pharmazeutische Formulierung gemäß Anspruch 17, wobei das zusätzliche aktive Ingredienz ein Corticosteroid ist.

19. Pharmazeutische Formulierung gemäß Anspruch 17, wobei das zusätzliche aktive Ingredienz ein antimuskarinisches/anticholinerges Arzneimittel ist.

20. Verwendung der pharmazeutischen Formulierung gemäß einem der vorangehenden Ansprüche für die Herstellung eines Medikaments zur Prävention oder Behandlung einer Erkrankung, die durch reversible Luftwegsobstruktion **gekennzeichnet** ist.

21. Verwendung gemäß Anspruch 20, wobei die Erkrankung Asthma oder chronische obstruktive Luftwegserkrankung (COPD) ist.

22. Kit, umfassend:
a) eine pharmazeutische Formulierung gemäß einem der Ansprüche 1 bis 19, gefüllt in Phiolen zur Einzeldosierungsverabreichung, und
b) ein Vernebelungsgerät.

## Revendications

1. Formulation pharmaceutique liquide et exempte de propulseur sous la forme d'une préparation prête à l'emploi pour une administration par nébulisation, comprenant :
i) un sel hydrosoluble physiologiquement acceptable de 8-hydroxy-5-[(1R)-1-hydroxy-2-[[(1R)-2-(4-méthoxyphényl)-1-méthyléthyl]amin]éthyl]-2(1H)-quinolinone (carmotérol) en tant que principe actif ;
ii) un solvant choisi parmi de l'eau ou une solution aqueuse comprenant au moins 50 % v/v d'eau et un co-solvant miscible à l'eau ;
iii) un tampon
dans laquelle le pH de la solution est compris entre 4,0 et 5,0 et le tampon comprend de l'acide citrique.

2. Formulation pharmaceutique selon la revendication 1, dans laquelle le sel de carmotérol est choisi dans le groupe constitué de sels avec des acides inorganiques, tels que les chlorhydrates, les bromhydrates, les phosphates, et les sulfates et de sels avec des acides organiques, tels que les salicylates, les citrates, les tartrates et les mandélates.

3. Formulation pharmaceutique selon la revendication 2, dans laquelle le sel est le chlorhydrate, désigné par CHF 4226.

4. Formulation pharmaceutique selon l'une quelconque des revendications 1 à 3, dans laquelle la concentration du sel hydrosoluble de carmotérol correspond à un pourcentage *p*/*v* de base exempte de carmotérol compris entre 0,0001 et 0,004 %.

5. Formulation pharmaceutique selon la revendication 4, dans laquelle la concentration du sel est comprise entre 0,0003 et 0,002 %.

6. Formulation pharmaceutique selon la revendication 5, dans laquelle la concentration du sel est comprise entre 0,0005 et 0,001 %.

7. Formulation pharmaceutique selon l'une quelconque des revendications précédentes, dans laquelle le pH de la solution est compris entre 4,0 et 4,5.

8. Formulation pharmaceutique selon l'une quelconque des revendications précédentes, dans laquelle le tampon est choisi dans le groupe constitué d'un couple acide citrique/citrate de sodium (tampon citrate) ou acide citrique/phosphate disodique.

9. Formulation pharmaceutique selon la revendication 8, dans laquelle le tampon est présent dans une concentration comprise entre 0,1 et 20 mM.

10. Formulation pharmaceutique selon la revendication 9, dans laquelle la concentration est comprise entre 1 et 15 mM.

11. Formulation pharmaceutique selon la revendication 10, dans laquelle la concentration est comprise entre 2 et 10 mM.

12. Formulation pharmaceutique selon l'une quelconque des revendications précédentes, dans laquelle le solvant ne comprend que de l'eau.

13. Formulation pharmaceutique selon la revendication 12, contenant de plus un agent d'ajustement de tonicité afin de fournir une osmolarité de l'ordre de 250 à 450 mOsm/l.

14. Formulation pharmaceutique selon la revendication 13, dans laquelle l'agent d'ajustement de tonicité est du chlorure de sodium.

15. Formulation pharmaceutique selon l'une quelconque des revendications 1 à 11, dans laquelle le co-solvant est un composé polaire qui contient un ou plusieurs groupes hydroxyle.

16. Formulation pharmaceutique selon la revendication 15, dans laquelle le composé polaire est du propylèneglycol.

17. Formulation pharmaceutique selon l'une quelconque des revendications précédentes, comprenant de plus un principe actif supplémentaire mis en suspension ou dissous dans le solvant.

18. Formulation pharmaceutique selon la revendication 17, dans laquelle le principe actif supplémentaire est un corticostéroïde.

19. Formulation pharmaceutique selon la revendication 17, dans laquelle le principe actif supplémentaire est un médicament antimuscarinique/anticholinergique.

20. Utilisation de la formulation pharmaceutique selon l'une quelconque des revendications précédentes pour la fabrication d'un médicament destiné à la prévention ou au traitement d'une maladie **caractérisée par** une obstruction des voies aériennes réversible.

21. Utilisation selon la revendication 20, dans laquelle la maladie est de l'asthme ou une broncopneumopathie chronique obstructive (« chronic obstructive airways disease » (COPD)).

22. Trousse comprenant :
a) une formulation pharmaceutique selon l'une quelconque des revendications 1 à 19 chargée dans des flacons pour une administration monodose ; et
b) un nébuliseur.
